(19) Europäisches Patentamt
European Patent Office
Office européen des brevets

(11) Publication number: **0 170 538**
**B1**

(12) **EUROPEAN PATENT SPECIFICATION**

(45) Date of publication of patent specification: **20.01.88**

(51) Int. Cl.$^4$: **A 23 K 1/16**

(21) Application number: **85401057.6**

(22) Date of filing: **29.05.85**

(54) **Use of certain compounds in livestock food as growth promotants.**

(30) Priority: **04.06.84 US 617299**
**08.05.85 US 731255**

(43) Date of publication of application:
**05.02.86 Bulletin 86/06**

(45) Publication of the grant of the patent:
**20.01.88 Bulletin 88/03**

(84) Designated Contracting States:
**BE CH DE FR GB IT LI LU NL SE**

(56) References cited:
**EP-A-0 048 626**
**US-A-3 974 277**

**CHEMICAL ABSTRACTS, vol. 81, no. 16, 21th October 1974, Columbus, Ohio, USA; C.A. BAILE et al. "Feeding elicited by alpha and beta adrenoceptor agonists in sheep and cattle", page 46, left column, abstract nr. 99544n & Pharmacol., Biochem. Behav., vol. 1, no. 5, 1973**

(73) Proprietor: **MERCK & CO. INC.**
**126, East Lincoln Avenue P.O. Box 2000**
**Rahway New Jersey 07065-0900 (US)**

(72) Inventor: **Muie, Larry A.**
**R.D. 3, Box 144**
**Flemington New Jersey 08822 (US)**

(74) Representative: **Ahner, Francis et al**
**CABINET REGIMBEAU 26, avenue Kléber**
**F-75116 Paris (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

STATEMENT OF THE INVENTION

This invention relates to a method of improving feed utilization and carcass composition by livestock which comprises the incorporation in the food or drinking water furnished to said livestock of from 0.01 to 100 parts per million of at least one compound selected from the group described in Table A. More specifically, it comprises a method of improving feed utilizatior and carcass composition in which the compounds of structures I, IV and IX are used. More specifically also, it relates to animal feed compositions for administration to livestock which comprises from 0.01 to 100 parts per million of a compound listed in Table A especially compounds I, IV and IX. The invention also relates to ruminants as slow release rumen boluses releasing doses of from 0.1 to 100 mg per head per day of at least one compound selected from the group listed in Table A especially compounds I, IV and IX. The invention also relates to all livestock as systemic implants that provide doses of 0.001 to 10 mg per head per day.

TABLE A

I. Zinterol

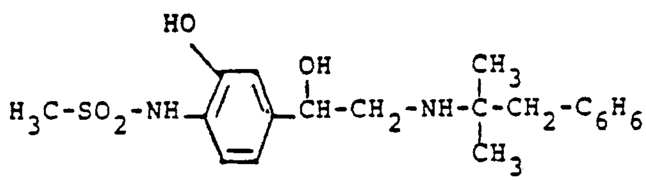

II. Z 1170

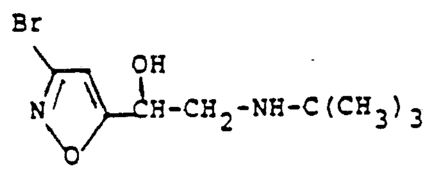

III. QH25B

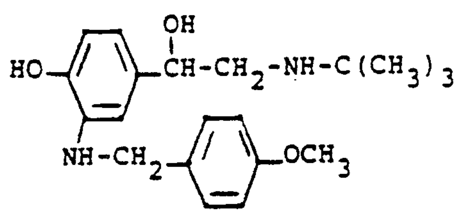

IV. L-644,969

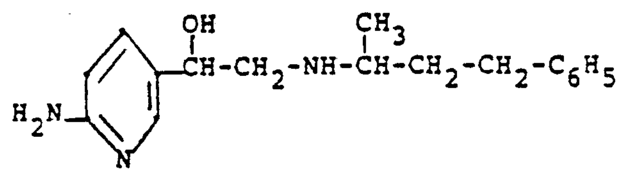

V. Bitolterol

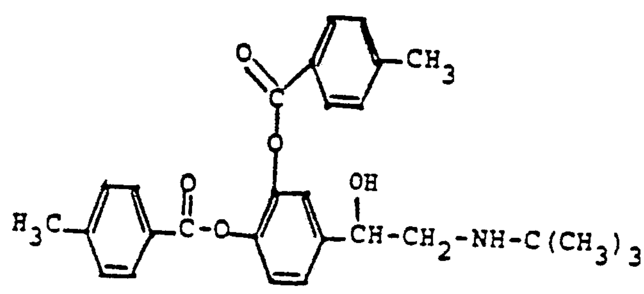

TABLE A CONT'D

VI. Reproterol 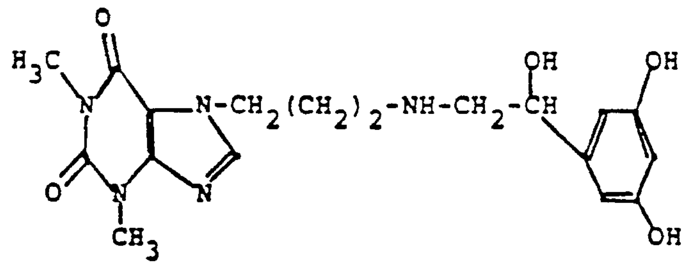

VII. Pirbuterol 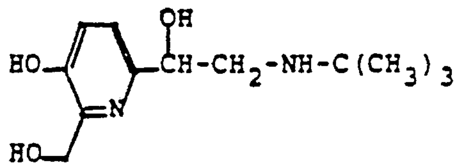

VIII. AA497 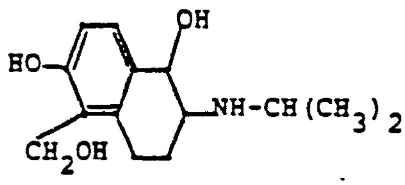

IX. Formoterol 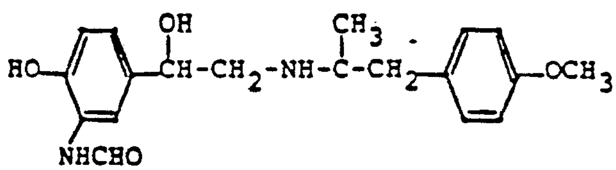

X. Colterol 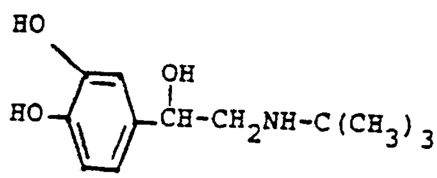

Zinterol, Bitolterol, Reproterol, Pirbuterol, Formoterol, Colterol are generic names. Z 1170, QH 25 B, L — 644,969, AA 497 are code names.

Compound X, Colterol, while similar to the other compounds in its growth promoting activity, differs in that very little activity is found when administered orally. It is however substantially active when administered parenterally.

It will be appreciated by those skilled in the art that the above compounds have one or more asymmetric centers and may exist as separable optical isomers or as equal mixtures of optical isomers which would be optically inactive racemic mixtures. The foregoing structures are presented in planar form, however, all possible optical isomers contained therein are intended to be included within the ambit of this invention.

One skilled in the art will also realize that separated optical isomers may often have different activities, one from the other, and such differences are also to be considered within the instant invention.

As an example of this, it is noted that compound IV has two centers of asymmetry, the first (referred to as "α") and third (referred to as "1") carbon atoms to the right of the pyridine ring carrying a hydroxy and a methyl substituent respectively. This will give rise to four possible optical isomers when the hydroxy and methyl are individually either above (β) or below (α) the plane of the molecule. In particular the isomer wherein both the hydroxy and methyl are β (which is more exactly referred to as the α-R,1R compound) has been found to be significantly more active than the most active of the other isomers.

BACKGROUND OF THE INVENTION

There is a need in animal husbandry for methods of improving the utilization of food. It is important not only that the growth of the animals be promoted but that the utilization of the food be directed in a different way, namely, to produce less fat and more protein. Some compounds have been described which enhance the utilization of food and change the carcass composition in some species of food livestock. Baker *et al.* have described (*Fed. Proc. 42 No. 4* p. 816, *No. 3* p. 668) that a compound called clenbuterol functions in this way. However, while this may be the best such agent published in the scientific literature, clenbuterol is not as active as other compounds in poultry, sheep or swine, especially compared to the compounds used in this invention.

DESCRIPTION OF THE INVENTION

We have found that the compounds listed in Table A are highly active in promoting the utilization of food by livestock intended for human consumption. They provide more growth and better utilization of the feed producing more protein and less fat. As can be seen in Table A the compounds involved differ widely in chemical structure. They also differ drastically from clenbuterol. While some of the active compounds listed in Table A are reported to have β-adrenergic activity, many compounds reported to be β-adrenergic agonists have very little or no growth promoting activity. Thus, accurate prediction of growth promoting activity on the basis of chemical structure or β-adrenergic activity is not possible.

Of a special use in this invention are the three compounds listed in Table A as I, IV, V and IX. All of these share high activity in all species under consideration. However, all of these compounds of Table A enhance the food utilization in some species by promoting growth and providing more protein and less fat for the same food intake. In particular, the high level of activity of Compounds V, bitolterol, combined with its very low level of toxicity, makes it a highly preferred growth promotant agent.

Example 1

IN VITRO TESTING

Adipose tissue collection and preparation.

Fat was collected from freshly killed animals as follows: epididymal and perirenal fat from 150 to 200 g male rats; perirenal fat from 20 to 25 kg wether lambs, and perirenal and omental fat from 35 to 50 kg gilts. Immediately after collection, fat was placed in a siliconized beaker (all glassware was siliconized) containing Krebs-Ringer bicarbonate solution (KRB; pH 7.4) at 37°C. Fat was transferred to a clean beaker and finely chopped with scissors. Chopped fat was then used as such (whole adipose preparation) or treated with collagenase (isolated adipocytes).

Isolation of adipocytes.

Chopped fat was placed in a shaker containing 70 ml cell dispersion medium (CDM; 5 mM glucose, .1% collagenase, 4% bovine serum albumin (BSA), in KRB) and incubated at 37°C for 1 hour with gentle shaking. Fat was then filtered to remove clumps and washed 4 times with washing medium (WM; 2.5 mM glucose, 1% BSA, in KRB) and 1 time with final suspension medium (FSM; 2.5 mM glucose, 2% essentially fatty acid free BSA, in KRB). Isolated adipocytes were then resuspended in the required amount of FSM (1 ml/flask, 3 flasks/treatment).

Determination of lipolytic activity in adipose tissue or adipocytes.

Whole adipose tissue from the rat (.3 ml) or isolated adipocytes from sheep or swine (1 ml) were placed in plastic scintillation vials containing FSM, (1.7 or 1 ml, respectively) and test compounds, and incubated at 37°C for 2 hours. Compounds were tested at concentrations of .01, .05, .25 and 1.25 μM. Isoproterenol, at the same concentrations, was used as a positive control. After incubation, media was removed and assayed for glycerol by a fluorometric modification of the method of Wieland (1974. Glucerol UV-method. pp. 1404. In: Bergmeyer (Ed.) Methods of Enzymatic Analysis. Verlag Chemie Weinham, Academic Press, New York).

Determination of anti-lipogenic activity in hepatocytes.

Hepatocytes were isolated from chicken liver using the procedure for adipocytes and incubated for 2 hours at 37°C in the presence of $^{14}C$ acetate and test compounds. Compounds were tested at concentrations of .01, .05, .25 and 1.25 μM. Isoproterenol, at the same concentrations, was used as a positive control. After incubation, 8 ml Doles reagent (isopropanol:hexane:$H_2SO_4$, 40:10:1) was added and incubation vials were shaken at room temperature for 30 minutes. Vial contents were poured into 50 ml screw-cap tubes. Vials were then rinsed with 6 ml hexane which was poured into the appropriate 50 ml tube. Tubes were shaken by hand, 10 ml $H_2O$ was added, and tubes were shaken again. After phase separation, 2 ml of the upper (hexane) phase was removed to scintillation vials. Hexane was evaporated by a gentle air flow and the remaining lipids were resuspended in 10 ml Scinti Verse II. $^{14}C$ was counted in a Packard liquid scintillation counter. Data are reported as CPM of $^{14}C$ acetate incorporated into total lipids.

Results of in vitro testing.

All nine compounds listed in Table A had significant antilipogenic activity. The three compounds identified as I, IV and IX appear to have five times the antilipogenic activity of isoproterenol and greater than 125 times the antilipogenic activity of clenbuterol in chicken hepatocytes. The nine compounds were tested for lipolytic activity in isolated swine (Table C) and ovine (Table D) adipocytes. All nine compounds significantly stimulated lipolysis in isolated swine or ovine adipocytes. Eight of the nine compounds were significantly more active than clenbuterol in stimulating lipolysis in isolated adipocytes. The lack of activity of clenbuterol in swine adipocytes should especially be noted.

The compounds were also tested (Table E) for lipolytic activity using chopped adipose tissue from rats and all nine compounds were found to be active. While rats are not a target species for the product, they are a good model for testing *in vivo*.

Example 2

In vivo testing

Eighty intact, male Charles River CD rats, 75—90 g, were housed and maintained on rat chow and water ad libitum. After four days acclimation ten rats per group were randomized by weight to treatment and pen. Compounds to be tested were premixed and added to ground rat chow using doses equivalent to 20 ppm of clenbuterol on a molecular weight basis. It had previously been determined 10 ppm of clenbuterol was the minimum dose needed to give maximum improvements in gain and feed conversion. Therefore, the results of any compound tested should cause responses similar to clenbuterol if the compound has half or more the activity of clenbuterol. The results are summarized in Table F and demonstrate that all nine compounds listed in Table A are capable of improving rate of gain and feed conversion and shifting carcass composition of growing animals from fat toward protein.

TABLE B

Comparative Activity of Inhibitors of Lipogenesis by Isolated Chicken Hepatocytes

(Measured by $^{14}C$ acetate incorporation in fatty acids and expressed as percent change compared to control)

| | LEVEL | | µM | |
|---|---|---|---|---|
| COMPOUND | 0.01 | 0.05 | 0.25 | 1.25 |
| Test 1 | | | | |
| Isoproterenol | −29.1 | −48.6 | −70.3 | −74.4 |
| Clenbuterol | −4.2 | −16.4 | −24.6 | −24.7 |
| I | −45.2 | −61.3 | −66.0 | −63.3 |
| IV | −33.7 | −54.3 | −63.2 | −65.1 |
| IX | −50.5 | −67.2 | −72.3 | −72.6 |
| III | −9.5 | −35.5 | −54.4 | −62.4 |
| V | 0 | −34.0 | −64.6 | −68.8 |
| VI | 0 | 0 | −8.6 | −31.2 |
| II | 0 | 0 | −7.9 | −18.4 |
| Test 2 | | | | |
| Isoproterenol | −21 | −15 | −49 | −61 |
| Clenbuterol | −8 | −18 | −16 | 1 |
| IX | −30 | −56 | −63 | −64 |
| VIII | 26 | −9 | −34 | −63 |
| VI | 3 | −12 | −25 | −28 |

TABLE C
Comparative Activity of Stimulators of Lipolysis by Isolated Swine Adipocytes

(Measured by glycerol production and expressed as percent increase compared to control)

| | LEVEL µM | | | |
|---|---|---|---|---|
| COMPOUND | 0.01 | 0.05 | 0.25 | 1.25 |
| Test 1 | | | | |
| Isoproterenol | 40 | 140 | 660 | 333 |
| Clenbuterol | 27 | −20 | −27 | −27 |
| I | 87 | 100 | 53 | 60 |
| IV | 260 | 333 | 80 | 53 |
| IX | 207 | 267 | 187 | 180 |
| VIII | 13 | 147 | 180 | 60 |
| V | 7 | 47 | 147 | 80 |
| III | 20 | 33 | 53 | 40 |
| VI | 20 | −7 | 0 | 140 |
| II | 27 | −7 | −40 | −27 |
| Test 2 | | | | |
| Isoproterenol | 175 | 196 | 820 | 800 |
| Clenbuterol | 0 | −20 | 4 | 17 |
| IX | 340 | 600 | 844 | 896 |
| VIII | 61 | 59 | 460 | 580 |
| VI | 8 | 21 | 3 | 160 |

TABLE D
Comparative Activity of Stimulators of Lipolysis by
Isolated Ovine Adipocytes

(Measured by glycerol production and expressed as
percent increase compared to control)

| | LEVEL μM | | | |
|---|---|---|---|---|
| COMPOUND | 0.01 | 0.05 | 0.25 | 1.25 |
| Test 1 | | | | |
| Isoproterenol | 670 | 1510 | 1820 | 2080 |
| Clenbuterol | inactive | 240 | 325 | 305 |
| I | 730 | 960 | 1775 | 2400 |
| IV | 820 | 1160 | 1540 | 1645 |
| IX | 675 | 695 | 1525 | 2400 |
| III | 465 | 845 | 1775 | 2255 |
| VIII | 135 | 445 | 565 | 720 |
| V | inactive | 435 | 1170 | 2140 |
| VI | inactive | 130 | 465 | 2010 |
| II | inactive | inactive | 325 | 180 |
| Test 2 | | | | |
| Isoproterenol | 600 | 1614 | 1800 | 2157 |
| Clenbuterol | 186 | 857 | 1171 | 1914 |
| IX | 2014 | 2100 | 2043 | 2271 |
| VIII | 71 | 1143 | 1800 | 2143 |
| VI | −14 | 129 | 700 | 1800 |

TABLE E
Comparative Activity of Stimulators of Lipolysis by Rat Adipose Tissue

(Measured by glycerol production and expressed as percent increase compared to control)

| | LEVEL µM | | | |
|---|---|---|---|---|
| COMPOUND | 0.01 | 0.05 | 0.25 | 1.25 |
| Test 1 | | | | |
| Isoproterenol | 45 | 173 | 290 | 272 |
| Clenbuterol | 12 | 26 | 21 | 15 |
| I | 90 | 129 | 281 | 334 |
| IV | 114 | 219 | 334 | 318 |
| IX | 42 | 91 | 258 | 322 |
| V | 17 | 33 | 111 | 288 |
| III | 194 | 203 | 334 | 288 |
| VI | 63 | 86 | 86 | 361 |
| II | 51 | 15 | 35 | 91 |
| VIII | 19 | 26 | 21 | 15 |
| Test 2 | | | | |
| Isoproterenol | 49 | 154 | 216 | 237 |
| Clenbuterol | 10 | 10 | 16 | 43 |
| IX | 16 | 70 | 145 | 222 |
| VI | 13 | 16 | 13 | 46 |
| VIII | −7 | 4 | 7 | 40 |

TABLE F
In vivo Testing in Rats

| Compound Test 1 | Dose | Weight g | Gain g/day | Feed Intake g/day | Feed Conversion g/day | Carcass Composition % of Change over Control Fat | Protein |
|---|---|---|---|---|---|---|---|
| Control | 0 | 191.8 | 7.3 | 18.6 | 2.55 | — | — |
| Clenbuterol | 10 | 198.6 | 7.8 | 19.2 | 2.48 | −11.3 | 11.2 |
| VII | 15.3 (a) | 200.9 | 8.0 | 19.8 | 2.48 | −8.7 | 3.6 |
| V | 38.5 (a) | 192.2 | 7.3 | 18.7 | 2.58 | −4.3 | 3.1 |
| III | 24.3 (a) | 201.7 | 8.0 | 19.4 | 2.41 | −7.0 | 6.1 |
| I | 26.4 (a) | 199.1 | 7.8 | 19.2 | 2.46 | −10.4 | 7.3 |
| IX | 21.8 (a) | 199.4 | 7.9 | 19.1 | 2.44 | −10.4 | 10.6 |
| VIII | 15.7 (a) | 202.6 | 8.1 | 19.5 | 2.41 | −6.1 | 6.4 |
| Test 2 Control | 0 | 202.3 | 7.5 | 19.3 | 2.63 | — | — |
| Clenbuterol | 10 | 216.9 | 8.2 | 19.9 | 2.46 | −11.9 | 4.3 |
| II | 19.0 (a) | 215.9 | 8.4 | 21.1 | 2.51 | −9.3 | 3.8 |
| VI | 27.0 (a) | 213.4 | 8.3 | 21.2 | 2.57 | 2.5 | 0.5 |
| IV | 23.0 (a) | 214.7 | 8.4 | 20.7 | 2.48 | −16.1 | 5.0 |

(a) Dose based on molecular weight equivalent of 20 ppm clenbuterol.

Example 3

Compound V (bitolterol) was compared to clenbuterol for effects on growth, feed conversion and carcass composition of broilers raised in floor pens for 7 weeks. Bitolterol at 0.5, 1.0 and 2.0 ppm in the diet iproved rate of gain, feed conversion and carcass protein in a dose-related manner. Bitolterol at the highest dose improved rate of gain 2.6% (P .01), feed conversion 3.8% (P .05) and carcass protein 4.9% (P .10). The later responses were better than those observed with clenbuterol at 0.5 ppm. Bitolterol at these doses did not reduce carcase fat.

Clenbuterol at 0.5 ppm in the diet was administered for 7 weeks to one group and only during the last 4 weeks to another group. Administration of clenbuterol for 7 weeks was slightly more effective for improving feed conversion and carcass protein than clenbuterol for 4 weeks. Clenbuterol given for 7 weeks improved feed conversion 3.5% (P .05) and appeared to improve (not significant) rate of gain 0.4%, carcass weight 0.7% and carcass protein 4.0% and to decrease (not significant) carcass fat 7.1%.

Dosage Utilization

The method of this invention is intended to increase the rate of body weight gain and/or feed efficiency and to decrease the carcass fat and increase the carcass protein of livestock such as cattle, sheep, poultry, pigs, etc. The compounds described herein Table A can be administered in doses of from 0.01 to 100 ppm in feed or drinking water. In addition, they may be administered to ruminants as slow release rumen boluses at doses of 0.1 to 100 mg per head per day or to all livestock as parenteral formulations such as systemic implants providing doses of 0.001 to 10 mg per head per day. The boluses and subcutaneous implants typically are designed to provide for continuous administration of the active growth promoting compound for about 100 days.

**Claims**

1. A method of improving the growth of livestock which comprises incorporating into the feed or drinking water furnished to said livestock from 0.01 to 100 parts per million of the active compound; into a ruminal bolus which provides from 0.1 to 100 mg per head per day of the active compound; wherein the active compound consists of at least one compound selected from Compounds I through IX inclusive as set forth in Table A below; or into a parenteral formulation which provides from 0.001 to 10 mg per head per day of the active compound wherein the active compound consists of at least one compound selected from Compounds I through X inclusive as set forth in Table A below.

TABLE A

I. Zinterol 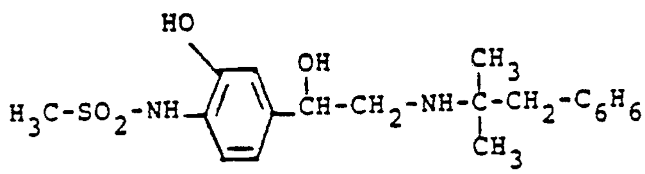

II. Z 1170 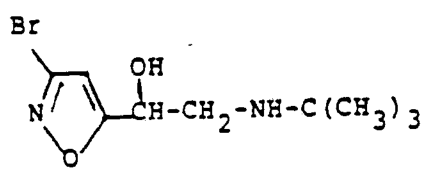

III. QH25B 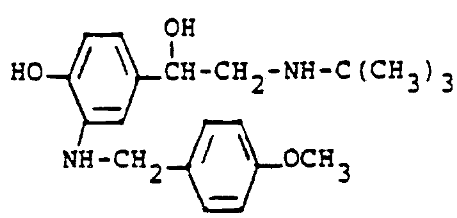

IV. L-644,969 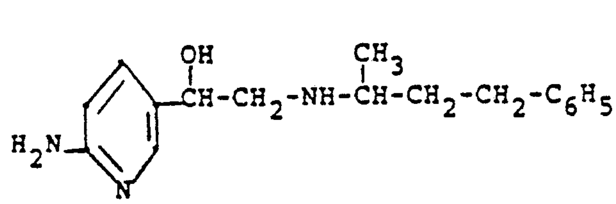

TABLE A CONT'D

V. Bitolterol

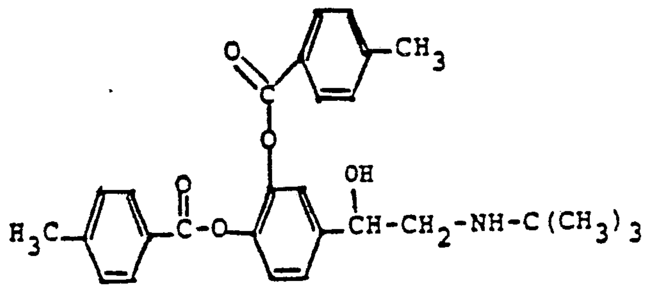

VI. Reproterol

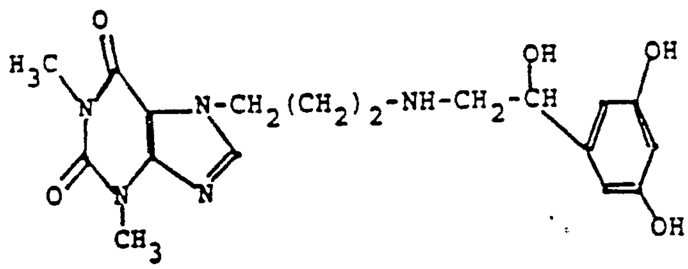

VII. Pirbuterol

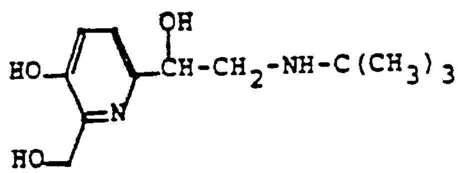

VIII. AA497

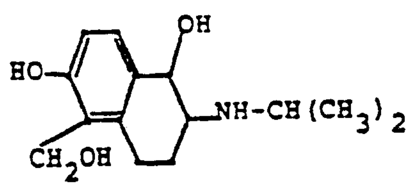

IX. Formoterol

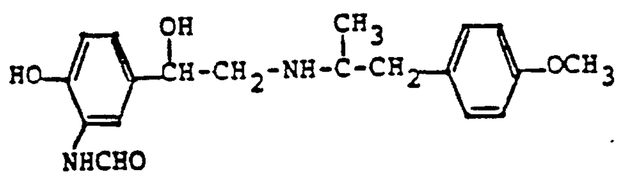

X. Colterol

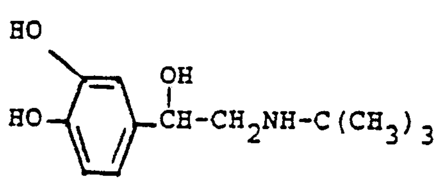

2. The method of Claim 1 in which the compounds are selected from the group consisting of compounds I, IV, V and IX.

3. The method of Claim 2 in which the compound is Compound I.

4. The method of Claim 2 in which the compound is Compound IV.

5. The method of Claim 1 in which the compound is Compound V.

6. The method Claim 2 in which the compound is Compound IX.

7. The method Claim 1 in which the compound is Compound II.

8. The method Claim 1 in which the compound is Compound III.

9. The method Claim 1 in which the compound is Compound VI.

10. The method Claim 1 in which the compound is Compound VII.

11. The method Claim 1 in which the compound is Compound VIII.

12. The method Claim 1 in which the compound is Compound X.

13. Animal feed compositions for administration to livestock which comprises 0.01 to 100 parts per million of at least one compound selected from the group of compounds described in Table A of Claim 1 as Compounds I through IX inclusive.

14. Animal feed compositions of Claim 13 which comprises at least one compound selected from the group consisting of Compounds I, IV, V and IX.

15. A slow release ruminal bolus capable of continuously administering from 0.1 to 100 mg per head per day of at least one compound selected from the group comprising those described in Table A of Claim 1 as Compounds I through IX inclusive.

16. The bolus of Claim 15 containing a compound selected from the group consisting of Compounds I, IV, V and IX.

17. A parenteral formulation capable of continuously administering from 0.001 to 10 mg per head per day of at least one compound selected from the group comprising those described in Table A of Claim 1 as Compounds I through X inclusive.

18. The parenteral formulation of Claim 17 which is a subcutaneous implant.

19. The subcutaneous implant of Claim 18 containing a compound selected from the group consisting of Compounds I, IV, V and IX.

**Patentansprüche**

1. Verfahren zur Förderung des Wachstums von Vieh durch Einbringen von 0,01 bis 100 ppm der aktiven Verbindung in das dem Vieh verabreichte Futter oder Trinkwasser; durch Einbringen in einen Pansenbolus, wodurch 0,1 bis 100 mg pro Kopf und Tag der aktiven Verbindung bereitgestellt werden; wobei die aktive Verbindung aus wenigstens einer Verbindung, ausgewählt aus den Verbindungen I bis IX einschließlich, wie in nachstehender Tabelle A aufgeführt, besteht; oder durch Einbringen in eine parenterale Formulierung, welche 0,001 bis 10 mg pro Kopf und Tag der aktiven Verbindung bereitstellt, wobei die aktive Verbindung aus wenigstens einer Verbindung, ausgewählt aus den Verbindungen I bis X einschließlich, wie in nachstehender Tabelle A dargestellt, besteht;

TABELLE A

I. Zinterol

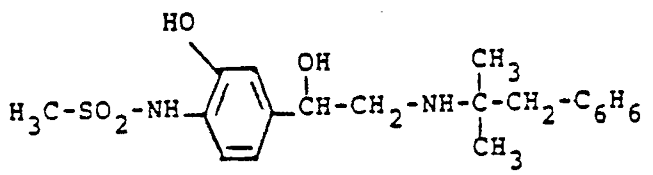

II. Z 1170

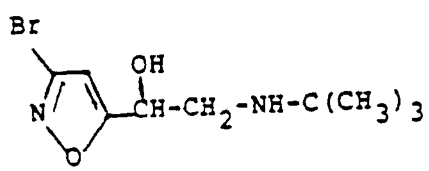

III. QH25B

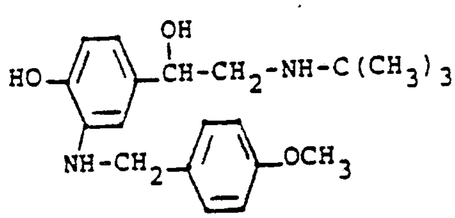

IV. L-644,969

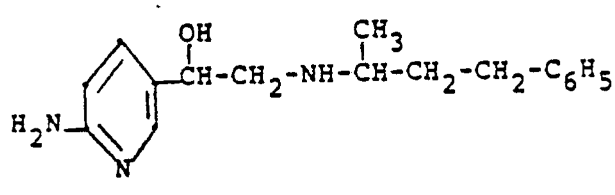

V. Bitolterol

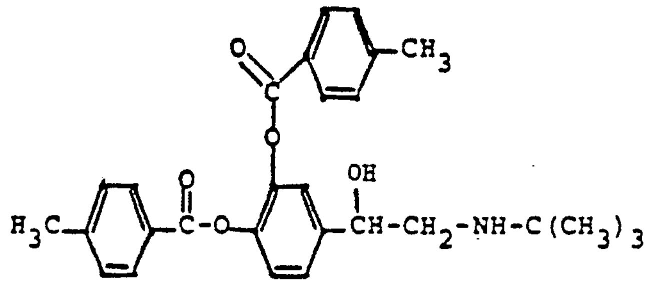

VI. Reproterol

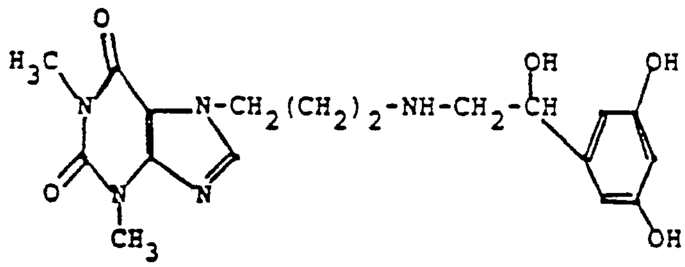

VII. Pirbuterol

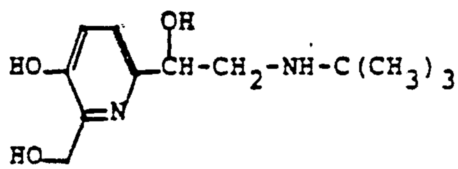

VIII. AA497

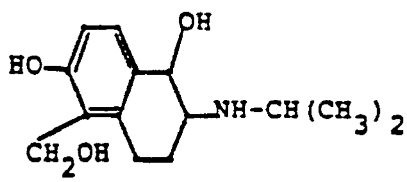

IX. Formoterol

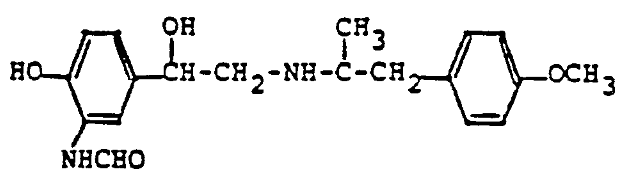

X. Colterol

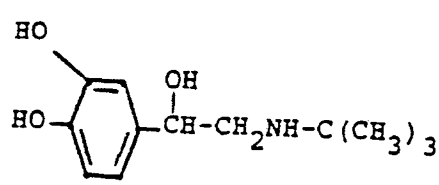

2. Verfahren nach Anspruch 1, worin die Verbindungen ausgewählt sind aus der Gruppe bestehend aus den Verbindungen I, IV, V und IX.

3. Verfahren nach Anspruch 2, worin die Verbindung die Verbindung I ist.

4. Verfahren nach Anspruch 2, worin die Verbindung die Verbindung IV ist.

5. Verfahren nach Anspruch 1, worin die Verbindung die Verbindung V ist.

6. Verfahren nach Anspruch 2, worin die Verbindung die Verbindung IX ist.

7. Verfahren nach Anspruch 1, worin die Verbindung die Verbindung II ist.

8. Verfahren nach Anspruch 1, worin die Verbindung die Verbindung III ist.

9. Verfahren nach Anspruch 1, worin die Verbindung die Verbindung VI ist.

10. Verfahren nach Anspruch 1, worin die Verbindung die Verbindung VII ist.

11. Verfahren nach Anspruch 1, worin die Verbindung die Verbindung VIII ist.

12. Verfahren nach Anspruch 1, worin die Verbindung die Verbindung X ist.

13. Viehfutterzusammensetzungen zur Verabreichung an Vieh, welche 0,01 bis 100 ppm wenigstens einer Verbindung, ausgewählt aus der in Tabelle A in Anspruch 1 als Verbindungen I bis IX einschließlich beschriebenen Gruppe von Verbindungen, enthalten.

14. Viehfutterzusammensetzungen nach Anspruch 13, welche wenigstens eine Verbindung, ausgewählt aus der Gruppe, bestehend aus den Verbindungen I, IV, V und IX, enthalten.

15. Pansenbolus mit verzögerter Freigabe, welcher in der Lage ist, kontinuierlich 0,1 bis 100 mg pro Kopf und Tag wenigstens einer Verbindung, die aus der Gruppe ausgewählt ist, die die in Tabelle A in Anspruch 1 als Verbindungen I bis IX einschließlich beschriebenen umfaßt, zu verabreichen.

16. Bolus nach Anspruch 15, enthaltend eine Verbindung, ausgewählt aus der aus den Verbindungen I, IV, V und IX bestehenden Gruppe.

17. Parenterale Formulierung, welche in der Lage ist, kontinuierlich 0,001 bis 10 mg pro Kopf und Tag wenigstens einer Verbindung, die aus der Gruppe ausgewählt ist, die die in Tabelle A in Anspruch 1 als Verbindungen I bis X einschließlich beschriebenen umfaßt, zu verabreichen.

18. Parenterale Formulierung nach Anspruch 17, welche ein subkutanes Implantat ist.

19. Subkutanes Implantat nach Anspruch 18, enthaltend eine Verbindung, ausgewählt aus der aus den Verbindungen I, IV, V und IX bestehenden Gruppe.

**Revendications**

1. Procédé d'amélioration de la croissance du bétail, qui consiste à incorporer dans l'alimentation ou l'eau fournie audit bétail de 0,01 à 100 ppm du composé actif; dans un bol pour rumen fournissant de 0,1 à 100 mg par tête et par jour du composé actif; dans lequel le composé actif consiste en au moins un composé choisi parmi les composés I à IX inclus qu'on présente dans le Tableau A ci-après; ou en une composition parentérale qui fournit de 0,001 à 10 mg par tête et par jour du composé actif, ce composé actif consistant en au moins un composé choisi parmi les composés I à X inclus que l'on présente dans le Tableau A ci-après:

TABLEAU A

I. Zintérol

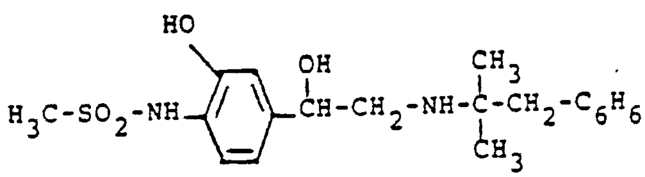

II. Z 1170

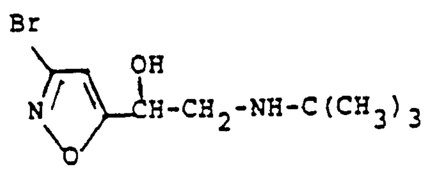

III. QH25B

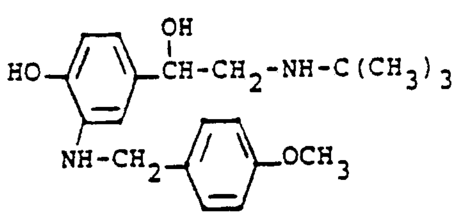

IV. L-644,969

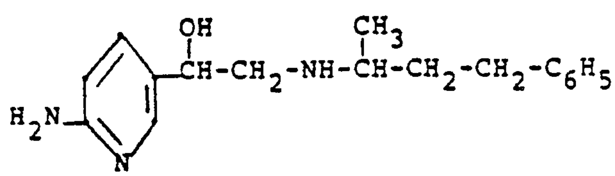

V. Bitoltérol 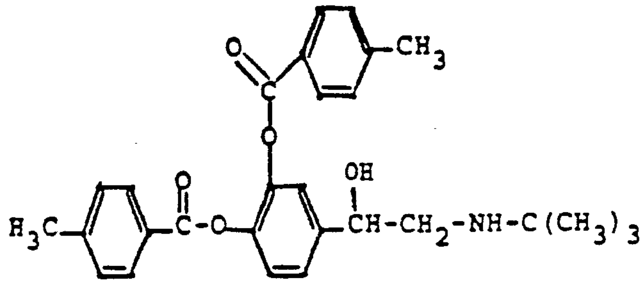

VI. Réprotérol 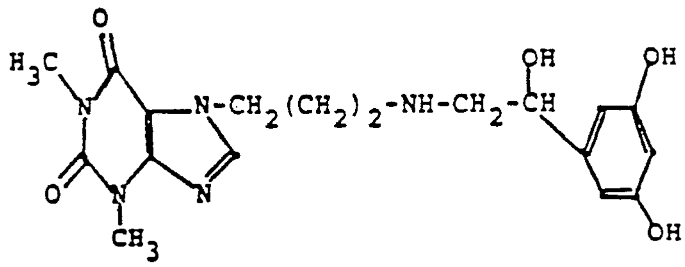

VII. Pirbutérol 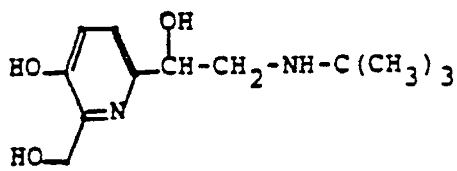

VIII. AA497 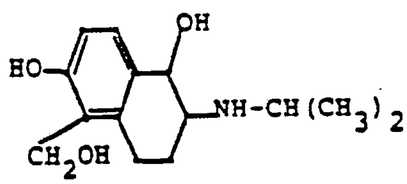

IX. Formotérol 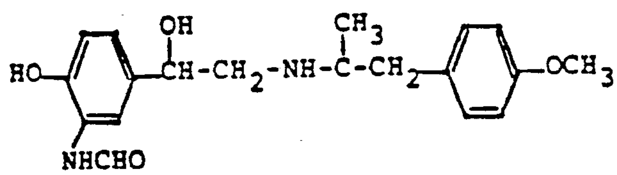

X. Coltérol 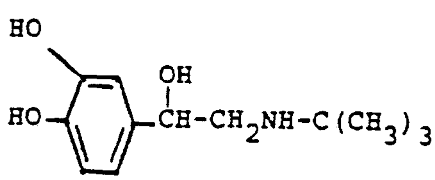

2. Procédé selon la revendication 1, dans lequel les composés sont choisis dans le groupe comprenant les composés I, IV, V et IX.

3. Procédé selon la revendication 2, dans lequel le composé est le composé I.

4. Procédé selon la revendication 2, dans lequel le composé est le composé IV.

5. Procédé selon la revendication 1, dans lequel le composé est le composé V.

6. Procédé selon la revendication 1, dans lequel le composé est le composé IX.

7. Procédé selon la revendication 1, dans lequel le composé est le composé II.

8. Procédé selon la revendication 1, dans lequel le composé est le composé III.

9. Procédé selon la revendication 1, dans lequel le composé est le composé VI.

10. Procédé selon la revendication 1, dans lequel le composé est le composé VII.

11. Procédé selon la revendication 1, dans lequel le composé est le composé VIII.

12. Procédé selon la revendication 1, dans lequel le composé est le composé X.

13. Composition d'alimentation animale pour administration au bétail, qui comprend 0,01 à 100 ppm d'au moins un composé choisi dans le groupe de composés décrits dans le Tableau A de la revendication 1, sous la dénomination des composés I à IX inclus.

14. Compositions d'alimentation animale selon la revendication 13, qui comprennent au moins un composé choisi dans le groupe comprenant les composés I, IV, V et IX.

15. Bol à libération lente pour rumen capable d'administrer en continu de 0,1 à 100 mg par tête et par jour d'au moins un composé choisi dans le groupe comprenant ceux décrits dans le Tablèau A de la revendication 1, sous la dénomination des composés I à IX inclus.

16. Bol selon la revendication 15, contenant un composé choisi dans le groupe comprenant les composés I, IV, V et IX.

17. Composition parentérale capable d'introduire en continu 0,001 à 10 mg per tête et par jour d'au moins un composé choisi dans le groupe comprenant ceux décrits dans le Tableau A de la revendication 1 sous la dénomination de composés I à X inclus.

18. Composition parentérale de la revendication 17 qui est un implant sous-cutané.

19. Implant sous-cutané de la revendication 18, qui contient un composé choisi parmi les composés I, IV, V et IX.